**Europäisches Patentamt**

**European Patent Office**

**Office européen des brevets**

(11) Veröffentlichungsnummer: **0 316 702**
**A2**

# (12) EUROPÄISCHE PATENTANMELDUNG

(21) Anmeldenummer: 88118506.0

(22) Anmeldetag: 07.11.88

(51) Int. Cl.⁴: **C12N 1/16 , C12P 7/44**

Der (Die) Mikroorganismus (Mikroorganismen) ist (sind) bei Deutsche Sammlung von Mikroorganismen unter der Nummer 4277 hinterlegt worden.

(30) Priorität: **16.11.87 DE 3738812**

(43) Veröffentlichungstag der Anmeldung:
**24.05.89 Patentblatt 89/21**

(84) Benannte Vertragsstaaten:
**AT BE CH DE ES FR GB GR IT LI LU NL SE**

(71) Anmelder: **Henkel Kommanditgesellschaft auf Aktien**
**Postfach 1100 Henkelstrasse 67**
**D-4000 Düsseldorf 1(DE)**

(72) Erfinder: **Kopp-Holtwiesche, Bettina, Dr.**
**Weststrasse 33**
**D-4000 Düsseldorf 13(DE)**
Erfinder: **Schindler, Joachim, Dr.**
**Am Eichelkamp 158**
**D-4010 Hilden(DE)**

(54) **Mikrobielle Synthese von Dodecandisäure.**

(57) Für ein Verfahren zur fermentativen Umwandlung von Fettsäureestergemischen, die zumindest anteilsweise Laurinsäureester enthalten, in Dicarbonsäuren mit gleicher C-Atomanzahl, bezogen auf den Fettsäureteil, soll ein Mikroorganismus zur Verfügung gestellt werden, der eine höhere Transformationsgeschwindigkeit für $C_{12}$-Verbindungen als für deren höhere Homologe aufweist. Dies gelang dadurch, daß die Umwandlung in Gegenwart eines Mikroorganismenstammes Candida tropicalis DC2-C2009 (DSM 4277) und/oder dessen Mutanten und/oder Varianten, die die Fähigkeit aufweisen, Methyllaurat mit höherer Transformationsgeschwindigkeit als dessen höhere Homologe in alpha-omega-Dicarbonsäuren umzuwandeln, durchgeführt wird.

EP 0 316 702 A2

EP 0 316 702 A2

## Mikrobielle Synthese von Dodecandisäure

Die Erfindung liegt auf dem Gebiet der spezifischen Umwandlung von $C_{12}$-Fettsäureestern zu alpha-omega-Dicarbonsäuren, und stellt für die gezielte Umwandlung von Methyllaurat neue Mutanten von Candida tropicalis sowie ein geeignetes Verfahren zur Verfügung.

Dicarbonsäuren mit mehr als 10 C-Atomen sind nach Methoden der präparativen organischen Chemie im technischen Maßstab schwer herstellbar. Dies gilt insbesondere für Dicarbonsäuren, die außer den beiden endständigen Carboxylgruppen noch weitere funktionelle Gruppen wie Doppelbindungen oder Hydroxylgruppen aufweisen.

Man hat daher versucht, aus gut zugänglichen Rohstoffen mit Hilfe der Stoffwechselleistungen verschiedener Mikroorganismen Dicarbonsäuren präparativ zu erzeugen. So wird in der deutschen Patentschrift 21 40 133 vorgeschlagen, auf Alkane, primäre Alkanole oder Carbonsäuren Hefen der Gattung Candida oder Pichia unter Fermentationsbedingungen einwirken zu lassen. Bei Einsatz von zum Beispiel einem speziellen Stamm von Candida lipolytica entstehen dabei Dicarbonsäuren, deren C-Kette gegenüber dem Ausgangsmaterial nicht oder nicht wesentlich abgebaut ist und auch ansonsten keine Veränderungen wie Einführen neuer funktioneller Gruppen aufweist. Ähnliche Verfahren, die von anderen Rohstoffen ausgehen, bei denen spezielle andere Mikroorganismen eingesetzt werden, sind beschrieben in den US-Patenten 3 975 234 und 4 339 536; in der britischen Patentschrift 1 405 026 sowie in den deutschen Offenlegungsschriften 21 64 626, 28 53 847, 29 37 292 und 29 51 177.

Das US-Patent 4 474 882 hat ungesättigte Dicarbonsäuren zum Gegenstand. Diese werden gewonnen, indem ein Stamm der Spezies Candida tropicalis zur Umwandlung von ungesättigten Monocarbonsäuren mit 14 bis 22 C-Atomen eingesetzt wird. Die ungesättigten Dicarbonsäuren entsprechen in der Anzahl und Lage der Doppelbindung den Ausgangsmaterialien.

Nach all den genannten Verfahren werden jedoch die gewünschten Dicarbonsäuren nur in recht kleinen Mengen erhalten. So sind im genannten US-Patent bei einer Fermentationsdauer von 2 Tagen Ausbeuten an Dicarbonsäure zwischen 0,65 g/l und maximal 1,96 g/l genannt. Für ein technisch genutztes Verfahren sind derartige Ausbeuten zu niedrig.

In der nicht vorveröffentlichten deutschen Patentanmeldung, Aktenzeichen P 37 21 119.6 wird eine Mutante von Candida tropicalis beschrieben, die in der Lage ist, Substrate mit einer Kettenlänge von 14 C-Atomen, beispielsweise Methylmyristat gezielt in eine Dicarbonsäure mit 14 C-Atomen umzuwandeln.

Vor dem Hintergrund dieses Standes der Technik bestand der Wunsch, einen Mikroorganismus zur Verfügung zu haben, der in der Lage ist, Ester der Laurinsäure, z.B. Methyllaurat, in die entsprechende Dicarbonsäure umzusetzen und der dabei eine höhere Transformationsgeschwindigkeit für $C_{12}$-Verbindungen als für deren höhere Homologen ($C_{14}$, $C_{16}$, $C_{18}$) aufweist. Darüber hinaus sollten für ein technisches Verfahren hinreichende Ausbeuten erreicht werden.

Gegenstand der Erfindung ist somit ein Candida tropicalis-Stamm mit der Hinterlegungsbezeichnung DSM 4277 (DC2-C2009) (hinterlegt nach Budapester Vertrag) sowie dessen Mutanten und Varianten, die die Fähigkeit aufweisen, Methyllaurat mit einer höheren Transformationsgeschwindigkeit als dessen höhere Homologe in alpha-omega-Dicarbonsäuren umzuwandeln.

Ein weiterer Gegenstand der Erfindung ist ein Verfahren zur fermentativen Umwandlung von Fettsäureestergemischen, die zumindest anteilsweise Laurinsäureester enthalten, in Dicarbonsäuren mit gleicher C-Atomanzahl, bezogen auf den Fettsäureteil, dadurch gekennzeichnet, daß die Umwandlung in Gegenwart eines Mikroorganismenstammes Candida tropicalis DC2-C2009 (DSM 4277) (hinterlegt nach Budapester Vertrag) und/oder dessen Mutanten und/oder Varianten, die die Fähigkeit aufweisen, Methyllaurat mit höherer Transformationsgeschwindigkeit als dessen höhere Homologe in alpha-omega-Dicarbonsäuren umzuwandeln, durchgeführt wird.

Der erfindungsgemäß eingesetzte Stamm Candida tropicalis DC2-C2009 (hinterlegt nach Budapester Vertrag) wurde durch Mutation und Selektion aus dem öffentlich zugänglichen Stamm Candida tropicalis ATCC 20336 gewonnen. Außer dem hinterlegten Stamme sind auch dessen Mutanten und Varianten einsetzbar, soweit sie die o.g. Fähigkeit zur bevorzugten Umwandlung von Laurinsäureestern zu Dodecandicarbonsäure besitzen.

Derartige Mutantenstämme lassen sich durch Mutation und Selektion, insbesondere durch mehrfache Mutation und mehrfache Selektion gewinnen. Die Mutation kann beispielsweise durch energiereiche Bestrahlung wie UV- oder Röntgenstrahlung erfolgen. Geeignet ist insbesondere aber auch die Behandlung der Zellen mit mutagenen Agentien. Geeignete mutagene Agentien sind beispielsweise Nitrosoguanidinverbindungen - wie beispielsweise 1-Methyl-3-nitro-1-nitrosoguanidin - oder Ethylmethansulfonat. Im einzelnen

2

kann hier auf die allgemeinen Angaben des Standes der Technik verwiesen werden, siehe hierzu beispielsweise US-Patentschrift 4 029 549, Spalte 2, Zeilen 57 ff., mit den dort enthaltenen Verweisungen.

Zur Herstellung von Mutanten, die für das erfindungsgemäße Verfahren geeignet sind, werden die Bedingungen von Konzentration und Einwirkzeit des mutagenen Agens so gewählt, daß die Ausgangspopulation durch die Behandlung zu 10 bis 99,999 Prozent inaktiviert wird. Vorzugsweise wird eine Abtötungsrate von 50 bis 99,9 Prozent gewählt.

Zur nachfolgenden Auslese der erfindungsgemäß geeigneten Mutanten aus der großen Population der Mikroorganismen nach der Mutationsbehandlung werden Kulturbedingungen gewählt, unter denen die abgeänderten spezifischen Eigenschaften der entstandenen Mutantenstämme zum Selektionsvorteil werden oder erkennbar werden. Zur Selektion kann die nach der Mutation erhaltene Mikroorganismenpopulation beispielsweise auf einem Minimalmedium angebrütet werden, das als einzige Kohlenstoffquelle die im erfindungsgemäßen Verfahren eingesetzten Substrate enthält. Diejenigen mutierten Mikroorganismen, die aufgrund der Mutationsbehandlung ihre Fähigkeit verloren haben, auf der jetzt vorliegenden Kohlenstoffquelle zu wachsen, können sich beim Bebrüten des Trennmediums nicht vermehren. Sie wachsen also nicht. Ein anderer Teil der Mutantenpopulation, der entweder bei der Mutationsbehandlung nicht hinreichend geschädigt worden ist oder andere Defekte erlitten hat, ist befähigt, auf der Kohlenstoffquelle des Trennmediums zu wachsen. Bei der Bebrütung tritt also eine Vermehrung ein. Um eine Trennung zu erreichen, enthält das Trennmedium Stoffe, die bewirken, daß die wachsenden Stämme aufgrund ihres Wachstums oder während ihres Wachstums abgetötet werden, ohne daß dabei die nicht wachsenden Mutantenstämme geschädigt werden. Möglich ist eine solche Trennung beispielsweise durch Zusatz von Antibiotika, wie z.B. Nystatin.

Eine andere Möglichkeit der Mutantentrennung in diesem Stadium ist der Einbau von zellschädigenden, insbesondere reaktiven Komponenten in den auf dem Trennmedium wachsenden Anteil der Mutantenpopulation. Geeignet ist beispielsweise der Einbau von $^{32}$P in die wachsenden Stämme. Möglich ist das beispielsweise durch Mitverwendung entsprechend radioaktiv markierter Salze der Phosphorsäure in der Nährlösung dieses Selektionsschritts. Für die Trennung mittels $^{32}$P hat sich insbesondere die Mitverwendung von $NaH_2{}^{32}PO_4$ in der Nährlösung bewährt.

Aus den somit angereicherten Mutanten können dann zum Beispiel mittels der an sich bekannten Lederbergschen Stempelmethode die erfindungsgemäß angestrebten Mutantenstämme (Defektblockmutantenstämme) isoliert werden. Bezüglich der hier eingesetzten Verfahrensweise und zur Antibiotika-Anreicherungsmethode und zur Lederbergschen Stempelmethode wird beispielsweise verwiesen auf J. Amer. Chem. Soc. 70, 4267, (1948) Davis, B.D. (Antibiotika-Anreicherungsmethode), J. Bact. 63, 399 (1952) Lederberg, J., Lederberg, E. M. (Lederbergsche Stempelmethode).

Zur weiteren Selektion der Mutanten wird der Fachmann deren Fähigkeit zur bevorzugten Umwandlung von Laurinsäureestern im Vergleich zu deren höheren Homologen berücksichtigen. Weiter berücksichtigen wird er die Menge der erzeugten Dicarbonsäuren. So kann erfindungsgemäß Dodecandisäure unter Schüttelkolbenbedingungen in Mengen von mindestens 10 g/l pro 96 Stunden Transformationszeit aus Methyllaurat hergestellt werden.

Die erfindungsgemäßen Mutanten setzen Laurinsäure selbst nicht zu Dodecandisäure um. Vielmehr wirkt Laurinsäure bereits in Konzentrationen von größer 0,1 Gew.-% zytotoxisch. Um so erstaunlicher für den Fachmann ist die hohe Transformationsleistung für Ester der Laurinsäure.

Als bevorzugte Ausgangsprodukte im erfindungsgemäßen Verfahren können die Ester laurinsäurereicher Fettsäuremischungen mit $C_1$ bis $C_6$-Alkoholen, insbesondere $C_1$ bis $C_6$-Monoalkoholen eingesetzt werden. So können insbesondere Methylester von Kokosöl oder Palmkernöl oder daraus durch Anreicherungs- und Destillationsverfahren hergestellte methyllauratreiche Schnitte eingesetzt werden. Ebenso geeignet sind voll synthetisch hergestellte Fettsäureestergemische, die zumindest anteilweise Ester der Laurinsäure enthalten. Aus diesen Gemischen wird bevorzugt Dodecandisäure gebildet.

Der Fachmann wird in jedem Falle darauf achten, daß der Gehalt an freier Laurinsäure so gering wie möglich eingestellt wird.

Eine Gruppe von Monocarbonsäureestern, die im erfindungsgemäßen Verfahren als Ausgangsstoffe eingesetzt werden können, sind die Ester von Carbonsäuren, die durch Oligomerisierung von Ethylen und entsprechende Umwandlung zugängig sind.

Im Hinblick auf den Alkoholteil der eingesetzten Ester kann gesagt werden, daß Ester von Alkoholen einer Funktionalität von 1 bis 4 und einer C-Atom-Anzahl von 1 - 6 geeignet sind. Bevorzugt sind jedoch die Ester von Monoalkoholen, insbesondere die Ester von kurzkettigen Monoalkoholen, wie Methanol oder Ethanol. Das zur Durchführung des erfindungsgemäßen Verfahrens eingesetzte Fermentationsmedium entspricht den für die Anzucht von Candida, insbesondere von Mikroorganismen der Spezies Candida tropicalis bekannten und beschriebenen Medien. Geeignete Fermentationsmedien enthalten die üblichen

3

Spurenelemente, eine Stickstoffquelle und darüber hinaus zusätzlich eine mit dem zu transformierenden Substrat nicht identische Kohlenstoffquelle. Unter Spurenelementen sind hier Salze der Kationen Ammonium, Kalium, Natrium, Magnesium, Calcium, Mangan, Zink, Eisen oder der Anionen Phosphat, Sulfat, Chlorid zu verstehen. Als Stickstoffquelle können neben anorganischen Verbindungen auch Pepton, Hefeextrakt oder Maisquellwasser eingesetzt werden.

Die Fermentationslösung enthält weiterhin als Kohlenstoffquelle ein Cosubstrat. Als Cosubstrat kann beispielsweise Natriumacetat eingesetzt werden. Weiterhin ist es möglich, daß als Cosubstrat übliche Zucker wie Glucose, Fructose, Maltose, Trehalose und dergleichen eingesetzt werden. Besonders bevorzugte Cosubstrate sind Glucose und Glycerin.

Bei der Durchführung des Verfahrens hat es sich als zweckmäßig erwiesen, in zwei Stufen zu arbeiten. In der ersten Stufe werden die Mikroorganismen angezüchtet. Dabei kann es gewünscht sein, den pH auf Werte kleiner 7, beispielsweise auf einen Wert zwischen 5,5 und 7 einzuregeln und konstant zu halten und die Cosubstratmenge so zu wählen, daß Cosubstrate gegen Ende der ersten Stufe praktisch vollständig verbraucht ist.

In der zweiten Stufe kann der umzusetzende Fettsäureester hinzugefügt werden, wobei man allerdings zu Beginn der Stufe den pH auf Werte zwischen 7,1 und 8, insbesondere auf einen Wert um 7,2 einstellt. Es hat sich weiterhin als zweckmäßig erwiesen, in der zweiten Stufe nach einer Induktionsphase von mehreren Stunden, beispielsweise 4 bis 8 Stunden, so viel Cosubstrate zuzudosieren, daß die Zellatmung aufrecht erhalten bleibt. Der Fachmann wird hierzu mit einer geeigneten Elektrode den Sauerstoffgehalt des Fermentationsansatzes kontrollieren. Weiterhin hat es sich als zweckmäßig erwiesen, das umzuwandelnde Ausgangsprodukt während der Fermentationsdauer nach und nach zuzufügen.

Die erfindungsgemäß entstehenden Dicarbonsäuren können anteilsweise als Monoester vorliegen. Aus den Monoestern können die freien Säuren in an sich bekannter Weise durch Verseifen gewonnen werden.

Nach einer weiteren Ausführungsform des erfindungsgemäßen Verfahrens ist es bevorzugt, den zur Umwandlung vorgesehenen Ausgangsverbindungen zur besseren Verteilung im Fermentationsmedium Emulgatoren zuzufügen. So werden günstige Ergebnisse erzielt, wenn insgesamt 0,1 bis 3 Gew.-%, bezogen auf Fermentationsmedium, an Emulgatoren vorhanden sind. Geeignet sind hier physiologisch verträgliche Emulgatoren und insbesonders physiologisch verträgliche nichtionische Emulgatoren. So können beispielsweise Zuckerester oder auch Sorbitanester, ethoxylierte Zuckerester, ethoxylierte Sorbitanester oder auch Alkylglycoside eingesetzt werden.

Als weitere Hilfsstoffe können bei dem erfindungsgemäßen Verfahren auch Schauminhibitoren zugesetzt werden. Bevorzugt sind bioverträgliche Schauminhibitoren, wie beispielsweise solche auf Basis Polypropylenglykol. Nötigenfalls können weitere übliche Hilfsstoffe zugesetzt werden.

Die nach dem erfindungsgemäßen Verfahren erhaltenen Dicarbonsäuren entsprechen in ihrer Kettenlänge dem Säureanteil der eingesetzten Ester. Aufgrund der höheren Substratspezifität für $C_{12}$-Ester entsteht Dodecandisäure (Dekandicarbonsäure) besonderer Reinheit. So kann aus technischem Methyllaurat die genannte Säure in 98 %iger Reinheit erhalten werden.

Für viele Anwendungen sind die bei der Fermentation hergestellten gesättigten Dicarbonsäuregemische direkt einsetzbar. So können die Gemische beispielsweise direkt zur Modifizierung von Polymeren, insbesondere Harzen eingesetzt werden.

Die folgenden Beispiele wurden mit dem Stamm Candida tropicalis DC2-C2009 (DSM 4277) durchgeführt.

## Beispiele

## Beispiel 1

## 1. Kulturbedingungen und Reaktionsführung

## 1.1 Stammhaltung

Malzextrakt-Schrägröhrchen sowie Lyophilisate

1.2 Schüttelkolben (100 ml Arbeitsvolumen)

1.2.1 Vorkultur I

24 stündige Inkubation einer Schrägröhrchenabimpfung in 100 ml YM-Bouillon (Yeast-Malt-Extrakt-Boullion) pH 6 (Fa. Difco) bei 30°C und 150 rpm auf der Schüttelmaschine

Vorkultur II

| Yeast-Nitrogen-Base (Difco) | 0,67 % |
|---|---|
| Natriumacetat | 0,3 % |
| Pepton | 0,3 % |
| Hefeextrakt | 0,5 % |
| Glucose | 2 % |
| gelöst in 0,1 M Phosphat-Puffer | |
| pH | 7,2 |

Ansatz 100 ml in 500 ml Erlenmeyer-Schikane-Kolben
Inokulum 10 ml Vorkultur I
Inkubation bei 30°C und 150 rpm Schüttelgeschwindigkeit bis zum vollständigen Verbrauch der Glucose (ca. 24 Stunden)

1.2.2 Transformationskultur

| Yeast-Nitrogen-Base (Difco) | 0,67 % |
|---|---|
| ethoxyliertes Sorbitan Monooleat | 0,2 % |
| Methyllaurat (technisch) | 4 % |
| Phosphat-Puffer 0,2 M | |
| pH | 8 |

Ansatz 50 ml in 500 ml Erlenmeyer-Schikane-Kolben
Inokulum 50 ml Vorkultur II
Reaktions-Ph Wert 7,2
Inkubation bei 30°C und 150 rpm Schüttelgeschwindigkeit Einhalten einer glucosefreien Induktionsphase (4 - 8 Stunden)
tägliche Zudosierung von 0,5 % Glucose nach Ende der Induktionsphase
tägliches Nachstellen des pH-Wertes auf 7,2
tägliche Probennahme

Nach 96 Stunden Transformationszeit betrug die Dodecandisäureausbeute 10 g/l.

Beispiel 2

2. Herstellung im Kleinfermenter

2.1 Kleinfermentation (1,5 l Arbeitsvolumen)

Gerätetyp Fa. Braun, Biostat E (Rührfermenter)

5

## 2.2 Vorkultur

24 stündige Inkubation einer Schrägröhrchenabimpfung in Würze-Bouillon (150 ml/Fa. Merck).

## 2.3 Fermenterkultur

### 2.3.1 Medium

| | |
|---|---|
| Glucose | 3 % |
| Hefeextrakt | 0,3 % |
| Maisquellwasser | 0,1 % |
| $(NH_4)_2SO_4$ | 0,2 % |
| $NH_4NO_3$ | 0,1 % |
| $NaH_2PO_4$ | 0,1 % |
| $K_2HPO_4$ | 0,15 % |
| $MgSO_4 \cdot 7H_2O$ | 0,05 % |
| KCl | 0,05 % |
| $MnCl_2$ | 0,05 % |
| $ZnSO_4 \cdot 7H_2O$ | 0,01 % |
| $FeSO_4 \cdot 7H_2O$ | 0,005 % |
| Biotin | 0,0001 % |
| ethoxyliertes Sorbitan Monooleat | 0,2 % |
| pH | 6,0 |

### 2.3.2 Prozeßführung

Belüftung 1 vvm, Rührgeschwindigkeit 900 rpm, Antischaummittel auf Basis Polypropylenglykol, automatische pH-Regelung, $pO_2$-Elektrode. Wachstumsphase bis zum vollständigen Verbrauch der Glucose (24 bis 30 Stunden).
pH Shift von 6,0 auf 7,2; gleichzeitig Zugabe von 2 % Methyllaurat
Substratzudosierung semi-kontinuierlich über die gesamte Fermentation.
Cosubstratzugabe (Glucose) je nach Bedarf der Zellatmung angepaßt, aber früheste Dosierung nach einer 4 bis 8stündigen Induktionsphase.
tägliche Probenahme

Nach 120 Stunden Transformationszeit betrug die Dodecandisäureausbeute 40 g/l.

## Beispiel 3

Zur Bestimmung der Selektivität der eingesetzten Mutanten wurde mit Hilfe des Stammes Candida tropicalis DC2-C2009 (DSM 4277) eine Reihe unterschiedlicher Substrate umgesetzt. Die folgenden Ausbeuten wurden erhalten:

| Kettenlänge | Alkan | Monosäure | Methylester |
|---|---|---|---|
| C12 | 2-3 g/l | 0 | 10-12 g/l |
| C13 | 1 g/l | 3 g/l | 2 g/l |
| C14 | 1 g/l | 4 g/l | 3 g/l |
| C15 | Spur | n.b. | n.b. |
| C16 | 0 | 0 | 1 g/l |
| n.b. = nicht bestimmt | | | |

## Ansprüche

1. Candida tropicalis-Stamm mit der Hinterlegungsbezeichnung DSM 4277 sowie dessen Mutanten und Varianten, die die Fähigkeit aufweisen, Methyllaurat mit einer höheren Transformationsgeschwindigkeit als dessen höhere Homologe in alphaomega-Dicarbonsäuren umzuwandeln.

2. Verfahren zur fermentativen Umwandlung von Fettsäureestergemischen, die zumindest anteilweise Laurinsäureester enthalten, in Dicarbonsäuren mit gleicher C-Atomanzahl, bezogen auf den Fettsäureteil, dadurch gekennzeichnet, daß die Umwandlung in Gegenwart eines Mikroorganismenstammes Candida tropicalis DC2-C2009 (DSM 4277) und/oder dessen Mutanten und/oder Varianten, die die Fähigkeit aufweisen, Methyllaurat mit höherer Transformationsgeschwindigkeit als dessen höhere Homologe in alpha-omega-Dicarbonsäuren umzuwandeln, durchgeführt wird.

3. Verfahren nach einem der Ansprüche 1 oder 2, dadurch gekennzeichnet, daß als Ausgangsprodukte die Ester laurinsäurereicher Fettsäuremischungen mit $C_1$-$C_6$-Alkoholen, vorzugsweise Monoalkoholen eingesetzt werden.

4. Verfahren nach einem der Ansprüche 1 bis 3, dadurch gekennzeichnet, daß als Ausgangsprodukte Methylester von Kokosöl oder Palmkernöl oder voll synthetisch hergestellte Mehtyllaurate-Mischungen und/oder deren Destillate oder Anreicherungsprodukte eingesetzt werden.

5. Verfahren nach einem der Ansprüch 1 - 4, dadurch gekennzeichnet, daß der Candida tropicalis-Stamm DC2-C2009 in einer ersten Stufe bis zum praktisch vollständigen Verbrauch des Cosubstrats bei einem konstanten pH-Wert kleiner 7, aber größer 5,5 angezüchtet wird, der pH-Wert sodann auf einen Wert zwischen 7,1 und 8 angehoben wird und sodann einoder mehrmals das Fettsäuregemisch zugesetzt wird, wonach man nach Verstreichen einer Induktionsphase von mehreren Stunden soviel Cosubstrat zudosiert, daß die Zellatmung aufrechterhalten bleibt.

6. Verfahren nach einem der Ansprüche 1 bis 5, dadurch gekennzeichnet, daß im Fermentationsmedium Emulgatoren, Schauminhibitoren und gewünschtenfalls weitere Hilfsstoffe zugesetzt werden.